(19) Europäisches Patentamt
European Patent Office
Office européen des brevets

(11) EP 3 027 634 B1

(12) EUROPEAN PATENT SPECIFICATION

(45) Date of publication and mention of the grant of the patent:
**21.03.2018 Bulletin 2018/12**

(21) Application number: **14744860.9**

(22) Date of filing: **30.07.2014**

(51) Int Cl.:
***C07H 17/08*** *(2006.01)*

(86) International application number:
**PCT/EP2014/066422**

(87) International publication number:
**WO 2015/014907 (05.02.2015 Gazette 2015/05)**

# (54) PROCESS FOR PREPARATION OF TULATHROMYCIN

VERFAHREN ZUR HERSTELLUNG VON TULATHROMYCIN

PROCÉDÉ DE PRÉPARATION DE TULATHROMYCINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.07.2013 SI 201300211**

(43) Date of publication of application:
**08.06.2016 Bulletin 2016/23**

(73) Proprietor: **Farma GRS, d.o.o.**
**8000 Novo mesto (SI)**

(72) Inventors:
- **BERGANT SIMONCIC, Ana**
  **8210 Trebnje (SI)**
- **BURJA, Bojan**
  **8272 Zdole (SI)**
- **KLJAJIC, Alen**
  **3000 Celje (SI)**
- **PECAVAR, Anica**
  **8000 Novo mesto (SI)**
- **DOLENC, Tadej**
  **4226 Ziri (SI)**
- **PLEVNIK, Miha**
  **1295 Ivancna Gorica (SI)**

(74) Representative: **Andrae | Westendorp**
**Patentanwälte Partnerschaft**
**Uhlandstraße 2**
**80336 München (DE)**

(56) References cited:
**EP-A1- 0 988 310 EP-A1- 1 131 331**

- **TIDWELL T T: "OXIDATION OF ALCOHOLS BY ACTIVATED DIMETHYL SULFOXIDE AND RELATED REACTIONS: AN UPDATE", SYNTHESIS, GEORG THIEME VERLAG, STUTTGART, DE, no. 10, 1 October 1990 (1990-10-01), pages 857-870, XP000160078, ISSN: 0039-7881, DOI: 10.1055/S-1990-27036**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 027 634 B1

## Description

FIELD OF THE INVENTION

The present invention relates to a novel process for the preparation of tulathromycin. More specifically, this invention relates to a process for preparation of the compound of formula (II) which is an important intermediate in the synthesis of tulathromycin.

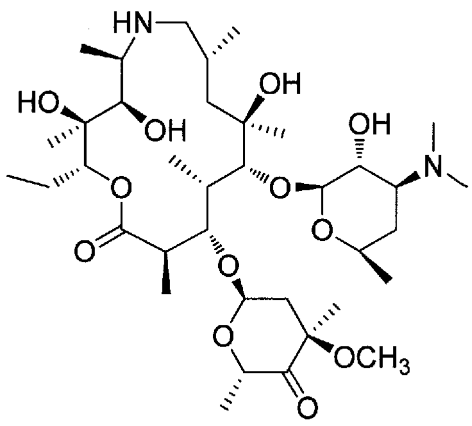

**(II)**

BACKGROUND OF THE INVENTION

Tulathromycin is a macrolide antibiotic used to treat bovine respiratory disease (BRD) in cattle and swine respiratory disease in pigs. It is marketed by Pfizer Inc. under the tradename Draxxin™. According to the EPAR-Scientific discussion document for Draxxin™, tulathromycin consists of two isomers, tulathromycin A (CP-472,295) and tulathromycin B (CP-547,272). Tulathromycin drug substance typically contains less than 1 % tulathromycin B, whereas tulathromycin drug product contains 8 to 13 % tulathromycin B. In solution, the two isomers form a stable equilibrated mixture which is considered as the active substance.

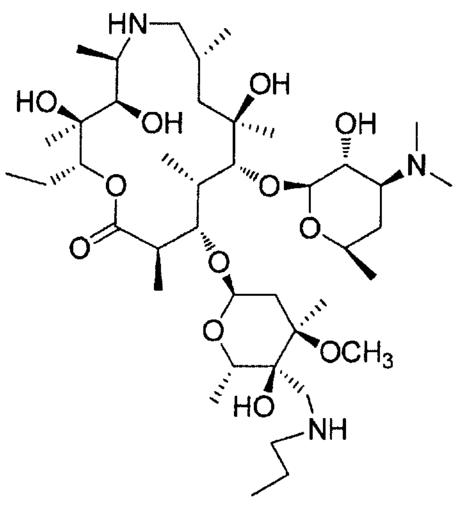

Tulathromycin A

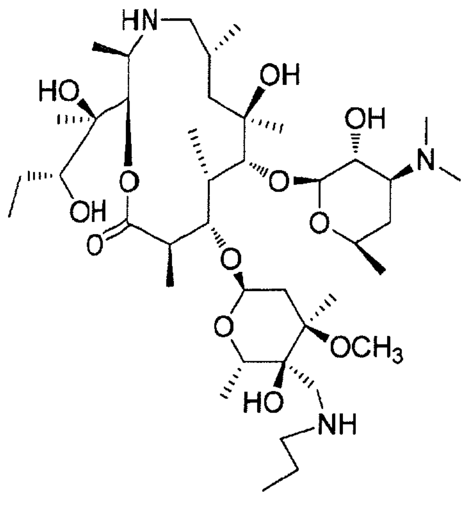

Tulathromycin B

Tulathromycin, its intermediates and processes for its preparation are disclosed in EP988310, EP1131331, WO02088158, EP1253153, WO2012038372, CN102295672 A, CN102260306 A, CN102786569 A, CN101648983 A, Letavic (Bioorg. Med. Chem. Lett., 2002, 12:2771-2774), Bronk (Bioorg. Med. Chem. Lett.,2003, 13:1955-1958), Wang (Applied Chemical Industry, 2012, 1294-1296), Xia (Editorial Office of Fine Chemicals, 2012, 795-799) and Xia (" Improving Process Of Synthesis Of Tulathromycin And Research Of Asymmetric Beckmann Rearranangement Reaction", 2013, Thesis).

Thomas T. Tidwell discusses in the publication "Oxidation of Alcohols by Activated Dimethyl Sulfoxide and Related Reactions: An Update", SYNTHESIS, No. 10, October 1990, the oxidation of alcohols by activated dimethyl sulfoxide. In particular, this publication mentions Swern procedure.

A common characteristic of prior art methods for the preparation of tulathromycin is protection of the C-2' hydroxyl group and optionally C-9a amino group prior to oxidation of the C-4" hydroxyl group of the compound of formula (I)

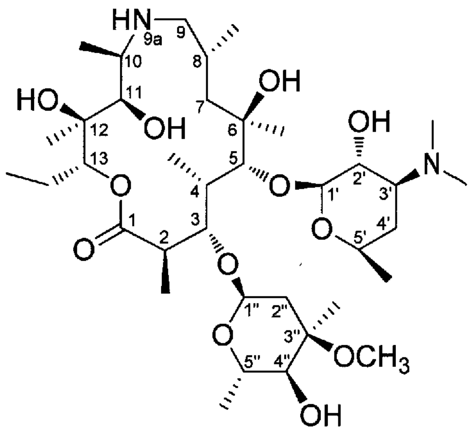

**(I)**

to form the compound of formula (A)

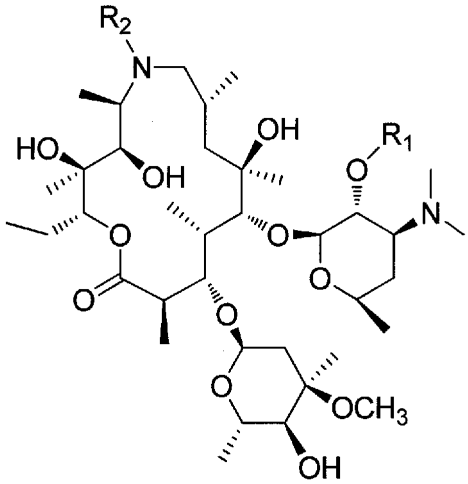

**(A)**,

wherein $R_1$ is a protecting group and $R_2$ is H or $R_1$. In prior art $R_1$ is $COOCH_2Ph$, $CH_3CO$ or $COOC(CH_3)_3$. The process disclosed in WO2012038372 uses the oxime derivative of formula (B)

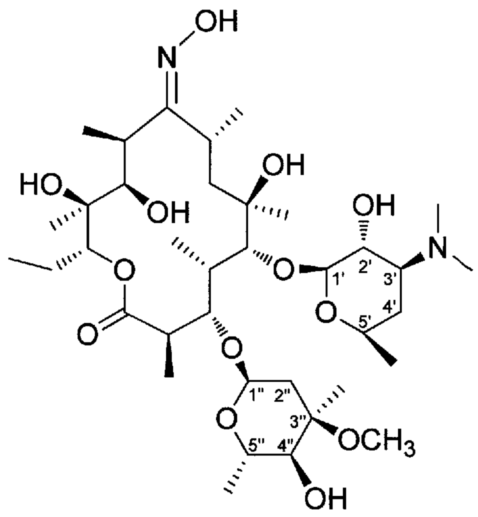

(B) ;

however, before the oxidation step the C-2' hydroxyl group and the oxime group are also protected to form the compound of formula (C)

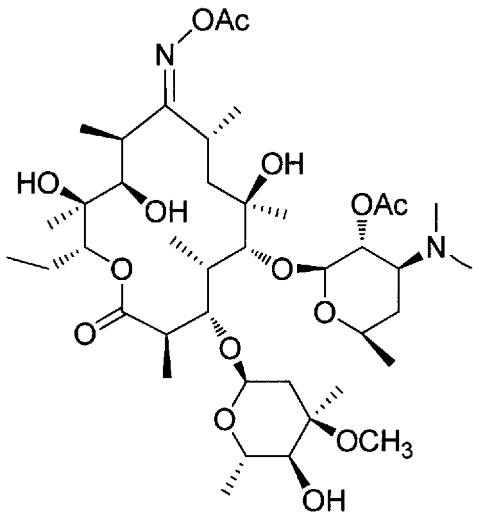

(C) .

The compounds of formula (A) and (C) are deprotected later on in the process for preparation of tulathromycin. It has been now surprisingly found that the compound of formula (II) can be prepared directly from the compound of formula (I) without protection of functional groups of the compound of formula (I). This is an improvement over prior art methods which require at least two additional steps due to protection and deprotection of C-2' hydroxyl group, thus prolonging the time and cost of the process, and very likely decreasing the yield. Furthermore, protection and/or deprotection of hydroxyl groups possess additional drawbacks, such as:

- use of harmful reagents and/or hazardous reaction conditions such as benzylchloroformate and/or hydrogenation with Pd/C;
- selective protection of only 1 or 2 functional groups in the molecule, and removal of one or two protecting groups;
- additional side reactions in protection and deprotection steps which result in more impurities;
- unstable intermediates;
- more waste due to more substances used.

BRIEF DESCRIPTION OF THE DRAWINGS

Figure 1 is an X-ray powder diffraction pattern of the compound of formula (I) as used in the Examples 1-3.

Figure 2 is an X-ray powder diffraction pattern of the compound of formula (II) prepared according to Example 6.
Figure 3 is an X-ray powder diffraction pattern of the compound of formula (III) prepared according to Example 14.
Figure 4 is an X-ray powder diffraction pattern of tulathromycin phosphate (1:2) prepared according to Example 16.

The X-ray powder diffraction patterns were obtained by PANalytical X' Pert PRO MPD powder diffractometer, X'celerator detector at CuKα radiation, 1.54178 Å, 3°<2θ<32.5°.

The purity of tulathromycin and its related substances is assessed by high pressure liquid chromatography (HPLC) with a column of the type X-Bridge Shield RP-18, (150 x 4.6)mm, 3 μm particles; detector: UV 200 nm; flow rate: 1.0 mL/min; injection volume: 10 μL; mobile phase: A: 0.01M phosphate buffer pH=10; B: mixture of acetonitrile and water (90:10); Gradient: 0'=45%B, 24'-33'=80%B, 35'-40'=45%B. This HPLC method is generally applicable for the analysis of tulathromycin and its related substances, chromatographic purity and assay. For assay analysis external standard method is used.

Detailed description of HPLC method for chromatographic purity and assay analysis:

Column: X-Bridge Shield RP-18, (150 x 4.6) mm, 3 μm particles,
Eluent A: 0.01 M $Na_3PO_4$ pH=10.0
Eluent B: acetonitrile/water = 90/10, (V/V)
Gradient:

| Time (min) | Eluent A (%) | Eluent B (%) |
|---|---|---|
| 0 | 55 | 45 |
| 24 | 20 | 80 |
| 33 | 20 | 80 |
| 35 | 55 | 45 |
| 40 | 55 | 45 |

Post time: 3 min
Flow-rate: 1.0 mL/min
Detection: UV, wavelength 200 nm
Injection volume: 10 μL
Column temperature: 25 °C
Diluent: acetonitrile

| Time (min) | % A | % B |
|---|---|---|
| 0 | 55 | 45 |
| 24 | 20 | 80 |
| 33 | 20 | 80 |
| 35 | 55 | 45 |
| 40 | 55 | 45 |

Sample preparation:

Accurately weigh about 50 mg of sample into 5 mL volumetric flask, dissolve and dilute to volume with diluent.

Calculation:

a) Chromatographic purity: Use area per cent method. Do not integrate solvent peaks.
b) Assay: Use external standard method.

$$\% \text{ substance} = \frac{Area_{sa} \times C_{st}}{Area_{st} \times C_{sa}} \times 100$$

Nuclear magnetic resonance (NMR) spectra were acquired using Varian VNMRS 400 MHz spectrometer at 25 °C. Samples were prepared as solutions in a suitable deuterated solvent and contained an internal calibration standard for assay determination.

DSC method:

Apparatus: DSC 1 Mettler Toledo
Heating rate: 10 °C/min
Gas: Nitrogen, 40 mL/min
Pan: Aluminium 40 μL with perforated lid
Sample mass: ~ 3 mg
Elemental analysis: Elemental analysis was performed on a Perkin Elmer 2400 Series II CHNS/O Analyzer.

DESCRIPTION OF THE INVENTION

The present invention relates to a process for the preparation of the compound of formula (II) wherein the compound of formula (I) is directly converted to the compound of formula (II) :

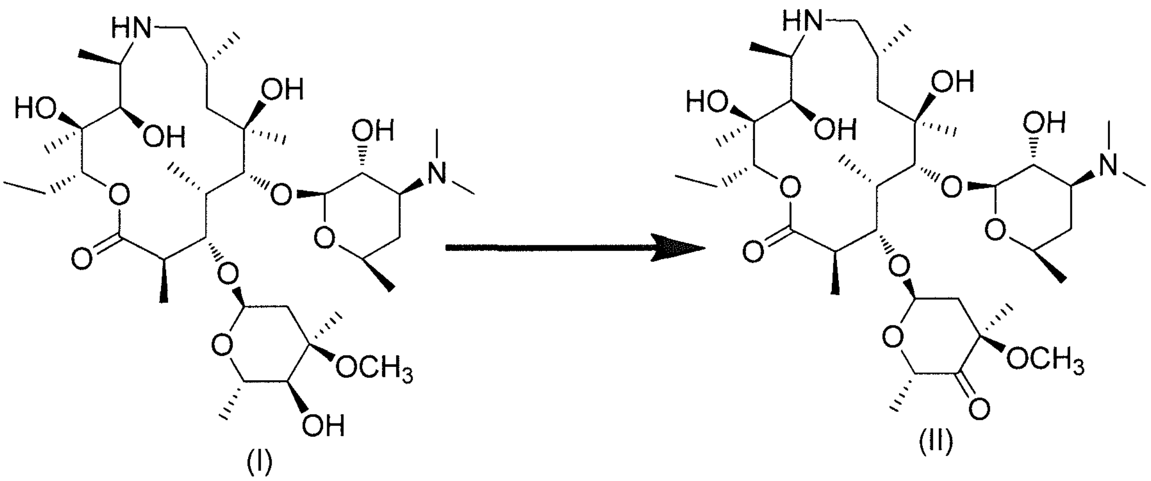

(I) (II)

The term "directly" according to the present invention means that the compound of formula (I) is converted to the compound of formula (II) without prior protection of the C-2' hydroxyl group or any other functional group of the compound of formula (I)

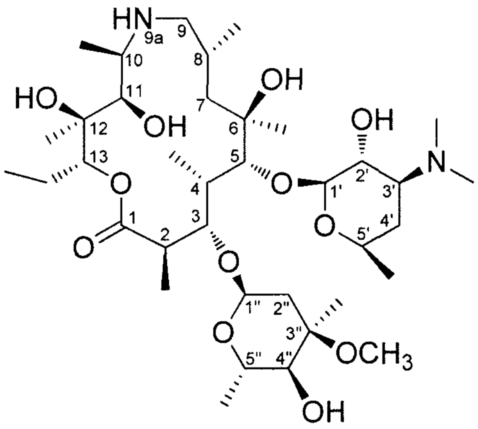

(I).

According to an embodiment, the term "directly converting compound of formula (I) to the compound of formula (II)" can be subjecting compound of formula (I) to an oxidation reaction with oxidant to convert compound of formula (I) to the compound of formula (II). Compound of formula (II) being obtained/being obtainable from subjecting compound of formula (I) to an oxidation reaction with oxidant.

The conversion of the compound of formula (I) to the compound of formula (II) is an oxidation process wherein the C-4" hydroxyl group is converted to a keto group. The oxidant is DMSO.

The conversion of the compound of formula (I) to the compound of formula (II) is performed in the presence of an appropriate activating agent. The activating agent is TFAA.

The conversion can be performed in the presence of a base, preferably an organic base, more preferably an organic amine such as diisopropylethylamine, and/or triethylamine ($Et_3N$). Most preferably, the base is triethylamine.

The solvent used in this conversion can be any suitable solvent such as a solvent selected from hydrocarbons, halocarbons, ethers, ketones, esters and/or nitriles. According to an embodiment, the solvent can be selected from the group consisting of hydrocarbons, halocarbons, ethers, ketones, esters, nitriles and/or mixtures thereof. Preferably, the solvent has a melting point below -80 °C. More preferably, the solvent is selected from the group of dichloromethane ($CH_2Cl_2$), toluene, tetrahydrofuran (THF), 2-methyltetrahydrofuran, diethyl ether, and/or acetone. Most preferably, the solvent is $CH_2Cl_2$. Preferably, the concentration of Compound (I) in $CH_2Cl_2$ is in the range from about 10 to about 200 mg/mL.

The reaction temperature can be in the range from about -90 °C to about 25 °C. Preferably, the reaction temperature is in the range from about -85 °C to about -30 °C, more preferably in the range from about -80 °C to about -30 °C.

In particular, the temperature during the step of subjecting compound of formula (I) to an oxidation reaction with oxidant can be in the range from about -90 °C to about 25 °C, preferably in the range from about -85 °C to about -30 °C, more preferably in the range from about -80 °C to about -30 °C.

According to an embodiment, the conversion of the compound of formula (I) to the compound of formula (II) comprises

a) dissolving the compound of formula (I) in a solvent, preferably in $CH_2Cl_2$, to form a solution comprising compound of formula (I), and optionally cooling the solution to a temperature of -80 to -30 °C,

b) adding an oxidant for converting compound of formula (I) to compound of formula (II), the oxidant being dimethyl sulfoxide (DMSO), to the solution comprising compound of formula (I) to form a reaction mixture, and optionally cooling the reaction mixture to a temperature of -80 to -30 °C,

c) carrying out a step selected from the group consisting of:

step c-1) adding an activating agent, the activating agent being TFAA, and adding a base, preferably an organic amine, more preferably $Et_3N$, to the reaction mixture,

step c-2) adding a base, preferably an organic amine, more preferably $Et_3N$, to the reaction mixture,

step c-3) adding an activating agent, the activating agent being TFAA, to the reaction mixture.

The temperature of the reaction mixture during the step of adding an activating agent or of adding an activating agent and a base can be in the range of -80 to -30 °C. The temperature of the reaction mixture during the step of adding a base can be in the range of -80 to -30 °C. Adding a base can be carried out simultaneously with or subsequently to adding an activating agent. Step c-1) or c-2) or step c-3) can be carried out simultaneously with step b) or after step b) of adding an oxidant. If necessary, the reaction mixture can be allowed to react after step b) or step c) or both step b)

and c). The reaction mixture may be subjected to further optional steps after above-indicated step c):

d) optionally contacting the reaction mixture with water to obtain an aqueous mixture, and
e) optionally isolating compound of formula (II) from the reaction mixture or from the aqueous mixture formed in optional step d).

Optionally, the compound of formula (I) can be added to the solution of an oxidant and an activating agent followed by addition of a base.

Preferably, the conversion of the compound of formula (I) to the compound of formula (II) is performed with DMSO, TFAA, and $Et_3N$ in $CH_2Cl_2$ at -80 to -30 °C. The reagents can be added into the reaction vessel in any order, more preferably, the conversion is performed with the addition of DMSO to a solution of the compound of formula (I) in $CH_2Cl_2$, followed by the addition of TFAA and $Et_3N$ at -80 to -30 °C. The reagents can be added into the reaction vessel in pure form or as a solution in a suitable solvent such as $CH_2Cl_2$.

The molar ratio of DMSO to the compound of formula (I) can be in the range from about 1 to about 40. Preferably, the molar ratio is in the range from about 15 to about 30, most preferably the ratio is in the range from about 20 to about 28.

The molar ratio of TFAA to the compound of formula (I) can be in the range from about 1.0 to about 4.0. Preferably, the molar ratio is in the range from about 1.5 to about 3.5, most preferably the ratio is in the range from about 2.0 to about 3.0.

The molar ratio of $Et_3N$ to the compound of formula (I) can be in the range from about 1.0 to about 10.0. Preferably, the molar ratio is in the range from about 3.0 to about 8.0, most preferably the ratio is in the range from about 4.0 to about 7.0.

If the compound of formula (II) prepared according to process of the present invention is not pure enough, it can be further purified by crystallization. The compound of formula (II), which can be in the form of a free base or in the form of hydrates, or in the form of a salt, can be crystallized from a solution in a suitable solvent, by utilizing a suitable solvent/antisolvent system or by suspending the material in a solvent having suitable compound (II) solubility characteristics at a predetermined temperature and stirring the suspension for a certain time. Such purifications can be performed at temperatures in the range from about -40 °C to about 200 °C. The (anti) solvent used in the crystallization can be selected from the group consisting of C1-C6 ketones such as methyl ethyl ketone, methyl isobutyl ketone, acetone and/or mixtures thereof; C1-C5 chlorinated hydrocarbons such as dichloromethane ($CH_2Cl_2$), chloroform, dichloroethane and/or mixtures thereof; C5-C10 alkanes or cycloalkanes such as hexane, heptane, octane, cyclohexane, cycloheptane and/or mixtures thereof; C1-C6 alcohols such as methanol, ethanol, 1-propanol, 2-propanol, 1-butanol, 1-pentanol, 2-pentanol, 1-hexanol and/or mixtures thereof; ethers such as diethyl ether, diisopropyl ether, methyl tert-butyl ether, tetrahydrofuran, 2-methyltetrahydrofuran and/or mixtures thereof; nitriles such as acetonitrile, propionitrile, butyronitrile, benzonitrile and/or mixtures thereof; and/or mixtures thereof. Preferably, the (anti)solvent is $CH_2Cl_2$, diethyl ether, acetone and/or mixtures thereof. Most preferably, the compound of formula (II) is purified by suspending the compound of formula (II) in acetone at a predetermined temperature and stirring the suspension for a certain time or by crystallization of the compound of formula (II) from a system consisting of $CH_2Cl_2$ and diethyl ether. Preferably, the predetermined temperature is in the range from about -20 °C to about 56 °C and the time for stirring is preferably in the range from about 10 min to about 24 h.

When compound of formula (II) is purified by crystallization from $CH_2Cl_2$/diethyl ether, the initial concentration of compound (II) in $CH_2Cl_2$ can be in the range from about 100 to 1000 mg/mL. Preferably, the concentration is in the range from about 200 to 900 mg/mL. Most preferably, the concentration is in the range from about 300 to 850 mg/mL.

When compound of formula (II) is purified by crystallization from $CH_2Cl_2$/diethyl ether, the V (CH2Cl2) / V (diethyl ether) ratio can be in the range from about 0.05 to about 0.8. Preferably, the ratio is in the range from about 0.1 to about 0.7. Most preferably, the ratio is in the range from about 0.2 to about 0.6.

When compound of formula (II) is purified by suspending the material in acetone at a predetermined temperature and stirring the suspension for a certain time, the ratio *m* (compound (II)) (mg) / V (acetone) (mL) can be in the range from about 50 mg/mL to about 1000 mg/mL. Preferably, the ratio is in the range from about 150 mg/mL to about 850 mg/mL. Most preferably, the ratio is in the range from about 200 to about 600 mg/mL. V being an abbreviation for volume, V (acetone) (mL) being an abbreviation for volume of acetone in mL, and m (compound (II)) (mg) being an abbreviation for mass of compound II in mg.

Described is a new crystalline form of the compound of formula (II) which is characterized by X-ray powder diffraction peaks at 8.7, 12.6, 15.7, 17.0, 17.7, 18.3, 19.7, 21.8, 23.5 and 24.4 $\pm$ 0.2° 2$\theta$. The X-ray diffractogram of the compound of formula (II) is depicted in Figure 2.

The compound of formula (I) as used herein can be prepared according to methods disclosed in prior art such as methods disclosed in GB2047247, EP109253, EP136831, HR9300886, EP259789, EP827965, EP879823, WO9902541, RU2144924, WO0100640, WO02009640, WO0244193, WO03102009, WO0215842, WO2007029266, WO2007017898, WO03102009, Marušić Išuk, Z. et al. Bioorg. Med. Chem. 2007, 15, 4498-4510.

The compound of formula (I) as used herein can have an HPLC assay of at least 50 %, preferably at least 80 % and most preferably at least 90 %.

The compound of formula (II) prepared according to the present invention can be used for the preparation of tulathromycin. The preparation of tulathromycin can be performed according to methods already disclosed in prior art such as prior art cited hereinabove. For example, the compound of formula (II) can be first converted to the compound of formula (III)

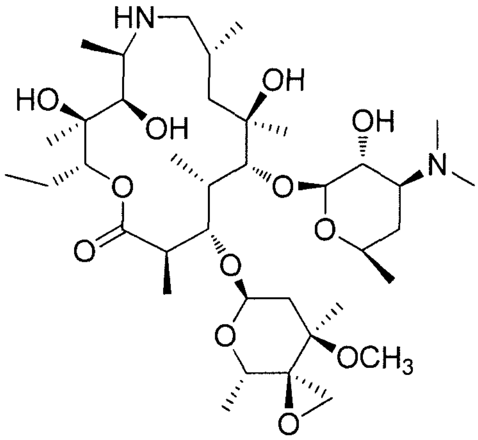

**(III),**

which can be in the form of a free base or in the form of hydrates, such as a monohydrate, or in the form of a salt, and then to tulathromycin or its pharmaceutically acceptable salts such as tulathromycin phosphate. The conversion from the compound of formula (II) to the compound of formula (III) can be performed by treating the compound of formula (II) with a suitable trimethylsulfonium salt, such as trimethylsulfonium tetrafluoroborate and/or trimethylsulfonium bromide, in the presence of a base, such as potassium tert-butoxide and/or its solution in a suitable solvent, such as tetrahydrofuran, and/or potassium hexamethyldisilazide and/or its solution in a suitable solvent, such as tetrahydrofuran. The conversion from the compound of formula (III) to tulathromycin or its pharmaceutically acceptable salts can be performed in a solvent selected from the group consisting of C1-C6 alcohols, such as 2-propanol, 1-propanol, 1-butanol, 2-butanol, *i*-butanol, 2-methyl-2-butanol, *t*-butanol, and/or mixtures thereof; ethers, such as diisopropyl ether, methyl *tert*-butyl ether, tetrahydrofuran, 2-methyltetrahydrofuran and/or mixtures thereof; C1-C5 chlorinated hydrocarbons such as dichloromethane ($CH_2Cl_2$), chloroform, dichloroethane and/or mixtures thereof; C5-C10 alkanes or cycloalkanes such as hexane, heptane, octane, cyclohexane, cycloheptane and/or mixtures thereof; nitriles such as acetonitrile, propionitrile, butyronitrile, benzonitrile and/or mixtures thereof; aromatic hydrocarbons, such as toluene, ethylbenzene and/or mixtures thereof; water; and/or mixtures thereof, such as *n*-heptane/2-propanol, diisopropyl ether/2-propanol; or under solventless conditions, where *n*-propylamine alone dissolves the compound of formula (III). Tulathromycin can be isolated in the form of free base or in the form of hydrates, such as a monohydrate, or in the form of its pharmaceutically acceptable salts or their hydrates, followed by conversion to tulathromycin base; or in crude form which is further subjected to purification, such as crystallization in the form of free base or in the form of hydrates, such as a monohydrate, or in the form of its pharmaceutically acceptable salts or their hydrates, followed by conversion to tulathromycin base. Tulathromycin or its hydrates, such as a monohydrate, or its salts or their hydrates can be in amorphous or crystalline form. Suitable crystalline forms are already known in prior art such as WO0069874, WO0102414, EP2402355 and WO2013013834. Tulathromycin or its hydrates, such as a monohydrate, or its salts or their hydrates can be further used for the preparation of pharmaceutically acceptable formulations, such as formulations disclosed in WO0155158, WO03011266, CN101647808, CN101933935 and WO2012001089.

According to an embodiment, the process for the preparation of the compound of formula (II) can be a process for the preparation of the compound of formula (II) and optionally for the preparation of a derivative thereof, said derivative being e.g. selected from tulathromycin, pharmaceutically acceptable salt(s) thereof, compound of formula (III), salt(s) of compound of formula (III), and hydrate(s) of compound of formula (III).

The process according to the present invention can be a process for the preparation of tulathromycin or a pharmaceutically acceptable salt thereof, which process further comprises: preparing tulathromycin or a pharmaceutically acceptable salt thereof from the compound of formula (II).

Furthermore, the process according to the present invention can be a process which further comprises: converting the compound of formula (II) into a compound of formula (III) or a salt or hydrate thereof,

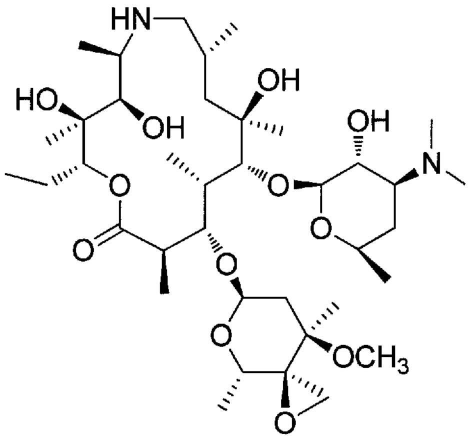

(III)

and optionally converting the compound of formula (III) or a salt or hydrate thereof to tulathromycin or a pharmaceutically acceptable salt or hydrate thereof.

The compound of formula (II) prepared according to the present invention can also be used for the preparation of the tulathromycin C-4" epimer according to methods described in prior art (EP988310) or by modifications of the methods described in prior art (EP988310), such as using potassium tert-butoxide as the base and/or THF as the solvent and/or lowering the temperature of the reaction.

Another object of the present invention is a process for preparation of tulathromycin wherein the compound of formula (III) is reacted with *n*-propylamine in a reaction mixture consisting of or comprising the compound of formula (III), n-propylamine and optionally water.

Compounds (I), (III), tulathromycin and/or their salts and/or their hydrates, such as monohydrates, can be purified by suspending the material in a solvent having suitable compound (I), (III), tulathromycin and/or their salts and/or their hydrates, such as monohydrates, solubility characteristics at a predetermined temperature and stirring the suspension for a certain time. Compounds (I), (III), tulathromycin and/or their salts and/or their hydrates, such as monohydrates, can also be purified by crystallization from a solution in a suitable solvent or by utilizing a suitable solvent/antisolvent system.

Another aspect of the invention concerns the use of the compound of formula (II) and the compound of formula (I) for the preparation of tulathromycin, wherein the compound of formula (II) is prepared directly from the compound of formula (I) without protection of functional groups of the compound of formula (I).

In particular, the present invention provides the following items:

1. A process for preparation of the compound of formula (II) wherein the compound of formula (I) is directly converted to the compound of formula (II):

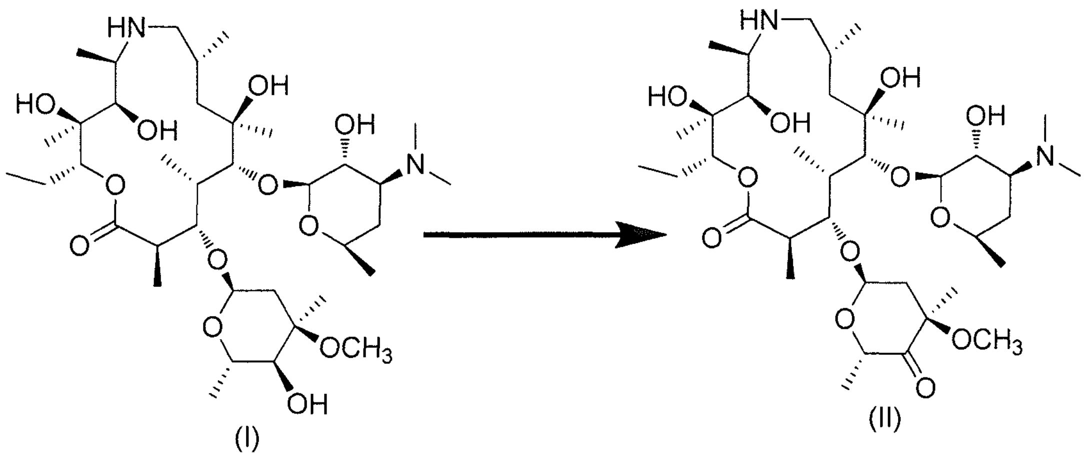

(I) (II)

,

wherein the conversion is performed with an oxidant and wherein the conversion is performed in the presence of an activating agent,
the oxidant being dimethyl sulfoxide, and the activating agent being trifluoroacetic anhydride.

2. The process according to item 1, wherein the compound of formula (I) is converted to the compound of formula (II) without prior protection of C-2' hydroxyl group or any other functional group of the compound of formula (I)

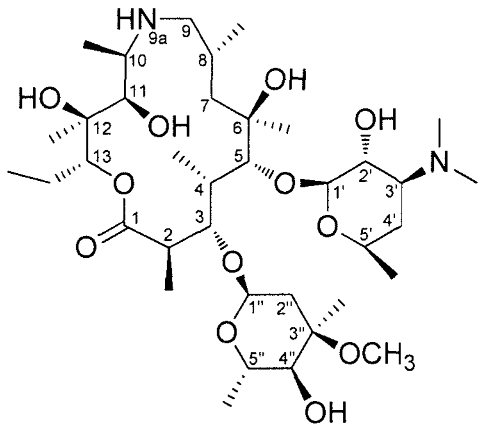

(I) .

3. The process according to any of items 1-2, wherein the conversion is performed in the presence of a base.

4. The process according to item 3, wherein the base is an organic amine.

5. The process according to item 4, wherein the base is triethylamine.

6. The process according to any of items 1-5, wherein the conversion is performed in a solvent which has a melting point below -80 °C.

7. The process according to item 6, wherein the solvent is dichloromethane, toluene, tetrahydrofuran, 2-methyltetrahydrofuran, diethyl ether, and/or acetone.

8. The process according to item 7, wherein the solvent is dichloromethane.

9. The process according to item 1, wherein the conversion is performed with dimethyl sulfoxide, trifluoroacetic anhydride and triethylamine in dichloromethane at -80 to -30 °C.

10. Use of the compound of formula (II) prepared according to any one of the preceding items, for the preparation of tulathromycin.

11. The process according to any of items 1-9, wherein purification of the compound of formula (II) comprises

a) suspending the compound of formula (II) in acetone at a predetermined temperature and stirring the suspension for a certain time; or
b) crystallizing the compound of formula (II) from a system consisting of $CH_2Cl_2$ and diethyl ether.

12. The process according to item 11, wherein the predetermined temperature is in the range from about -20 °C to about 56 °C and the time for stirring is in the range from about 10 min to about 24 h.

13. The process according to any of items 6 to 9, 11 to 12, wherein the solvent is solvent selected from the group consisting of dichloromethane, toluene, tetrahydrofuran, 2-methyltetrahydrofuran, diethyl ether, acetone, and mixtures thereof.

The present invention is illustrated with the following nonlimiting examples:

EXAMPLES

Example 1: Preparation of the compound of formula (II)

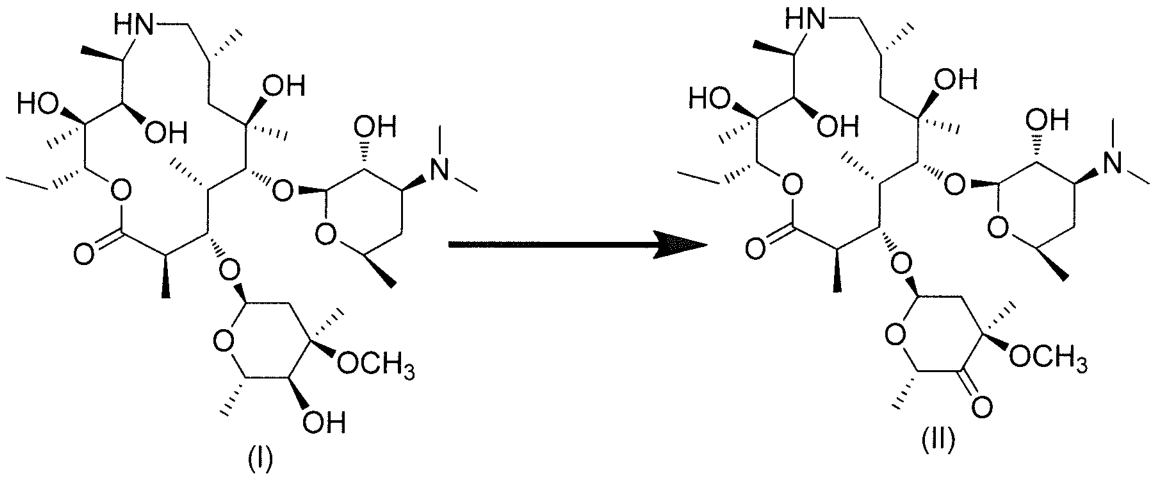

(I) (II)

80.1 g of the compound of formula (I) (HPLC assay 85 %) were dissolved in 1 L of $CH_2Cl_2$ and the solvent was removed under reduced pressure. The residue was redissolved in 1 L of fresh $CH_2Cl_2$ and 167 mL of anhydrous DMSO were added while stirring under a nitrogen atmosphere. After cooling to -78 °C, 38 mL of TFAA were added dropwise over a period of 15 min while stirring, with the temperature not exceeding -75 °C. The reaction mixture was stirred for additional 50 min at -78 °C. Next, 85 mL of $Et_3N$ were added dropwise over a period of 30 min with the temperature not exceeding -75 °C. The reaction mixture was stirred for additional 30 min at -78 °C. Next, 250 mL of $H_2O$ were added and the mixture was gradually warmed up to room temperature. Then it was stored at -20 °C overnight. The layers were separated and the organic phase was washed with 3 x 250 mL of $H_2O$. The solvent was removed under reduced pressure to afford 77.482 g (HPLC assay 72 %, yield 82 %) of the compound of formula (II).
HPLC purity: 71.05 area %

Example 2: Preparation of the compound of formula (II)

80.1 g of the compound of formula (I) (HPLC assay 85 %) were dissolved in 1 L of $CH_2Cl_2$ and the solvent was removed under reduced pressure. The residue was redissolved in 1 L of fresh $CH_2Cl_2$ and 167 mL of anhydrous DMSO were added while stirring under a nitrogen atmosphere. After cooling to -78 °C, 34 mL of TFAA were added dropwise over a period of 22 min while stirring with the temperature not exceeding -75 °C. The reaction mixture was stirred for additional 60 min at -78 °C. Next, 76 mL of $Et_3N$ were added dropwise over a period of 24 min with the temperature not exceeding -75 °C. The reaction mixture was stirred for additional 37 min at -78 °C. Next, 250 mL of $H_2O$ were added and the mixture was gradually warmed up to room temperature and stirred overnight. The layers were separated and the organic phase was washed with 3 x 250 mL of $H_2O$. The solvent was removed under reduced pressure to afford 79.8 g (yield 85 %) of material. After transfer and drying, 74.2 g (HPLC assay 74 %) of the compound of formula (II) were obtained.
HPLC purity: 70.79 area %

Example 3: Preparation of the compound of formula (II)

40.051 g of the compound of formula (I) (HPLC assay 85 %) were dissolved in 1 L of $CH_2Cl_2$ and the solvent was removed under reduced pressure. The residue was redissolved in 1 L of fresh $CH_2Cl_2$ and 84 mL of anhydrous DMSO were added while stirring under a nitrogen atmosphere. After cooling to -78 °C, a solution of 17 mL of TFAA in 17 mL of $CH_2Cl_2$ was added dropwise over a period of 55 min while stirring with the temperature not exceeding -78 °C. The reaction mixture was stirred for additional 70 min at -78 °C. Next, 38 mL of $Et_3N$ were added dropwise over a period of 11 min while stirring with the temperature not exceeding -76 °C. The reaction mixture was stirred for additional 165 min at -78 °C. Next, 250 mL of $H_2O$ were added and the mixture was gradually warmed up to room temperature over 17 h. The layers were separated and the organic phase was washed with 3 x 250 mL $H_2O$. The solvent was removed under reduced pressure and the product was vacuum dried at 35 °C for 9 h to afford 38.355 g (HPLC assay 80 %, yield 90 %) of the compound of formula (II).
HPLC purity: 72.92 area %

Example 4: Purification of the compound of formula (II)

81 g of the crude material (NMR assay 92 %) were dissolved in boiling $CH_2Cl_2$ (107 mL). Diethyl ether was added dropwise until crystallization began (130 mL). The heating was stopped and another 70 mL of diethyl ether were slowly added (approx. 20 min) to the mixture while stirring. The product was filtered off and vacuum dried to afford 62.232 g (NMR assay 98 %, yield 82 %) of the compound of formula (II), whose XRPD pattern is in accordance with that in Figure 2.

HPLC purity: 96.26 area %
DSC: $T_{onset}$ = 217.5° C
Elemental analysis: calculated for $C_{37}H_{68}N_2O_{12}$: C, 60.63; H, 9.35; N, 3.82; O, 26.19, found: C, 60.65; H, 9.52; N, 3.79.

Example 5: Purification of the compound of formula (II)

38.797 g of the crude material (HPLC assay 72 %), obtained in Example 1, were dissolved in boiling $CH_2Cl_2$ (46 mL). Diethyl ether was slowly added until crystallization began (130 mL). After this the heating was stopped and the stirring continued for another hour. The product was filtered off and vacuum dried to afford 20.479 g (HPLC assay 91 %, yield 67 %) of the compound of formula (II).
HPLC purity: 89.47 area %

Example 6: Purification of the compound of formula (II)

5.000 g of the crude material (HPLC assay 80 %), obtained in Example 3, were dissolved in 6.2 mL of $CH_2Cl_2$. 20 mL of diethyl ether were added dropwise over 80 min while stirring at room temperature. After 14 additional min of stirring at room temperature, the product was filtered off, washed with 7 mL of diethyl ether and dried under reduced pressure for 9 h at 35 C to afford 3.111 g (HPLC assay 94 %, yield 73 %) of the compound of formula (II).

HPLC purity: 91.61 area %
XRPD: Figure 2

Example 7: Purification of the compound of formula (II)

10 mL of acetone were added to 5.001 g of the crude material (HPLC assay 80 %), obtained in Example 3. The mixture was stirred at room temperature for approx. 140 min. The product was filtered off, washed with 7 mL of cold acetone and dried under reduced pressure for 9 h at 35 °C to afford 3.621 g (HPLC assay 95 %, yield 86 %) of the compound of formula (II), whose XRPD pattern is in accordance with that in Figure 2. HPLC purity: 92.27 area %

Example 8: Preparation of the compound of formula (III)

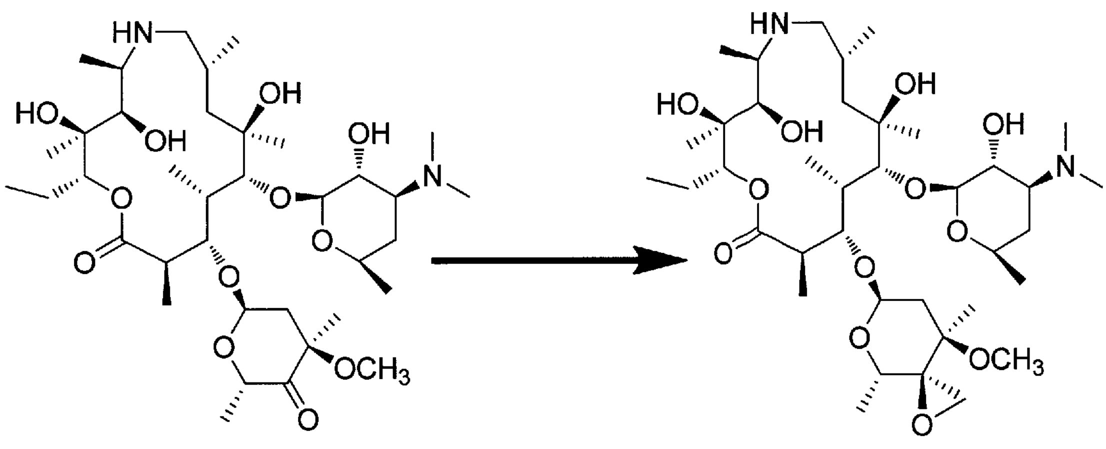

(II) (III)

A suspension of 6.50 g of $Me_3SBF_4$ in 100 mL of THF was cooled to -30 °C and 8.01 g of potassium tert-butoxide were added in one portion. The temperature reached -20° C in 20 min. The mixture was stirred under a nitrogen atmosphere at -22 to -18 °C for another 100 min. A solution of 5.00 g of the compound of formula (II) (HPLC assay 97 %) in 100 mL of $CH_2Cl_2$ was prepared and cooled to -78 °C under a nitrogen atmosphere.

The THF suspension was cooled to -78 °C and quickly added to the prepared $CH_2Cl_2$ solution while stirring under a nitrogen atmosphere. After additional 5 min of stirring at -78 °C, the reaction mixture was poured into a solution of $NH_4Cl$ (3.84 g) in $H_2O$ (31 mL). After stirring at room temperature for 60 min, the mixture was stored at -20 °C overnight. The layers were separated and the aqueous phase was washed with 2 x 8 mL $CH_2Cl_2$. The organic layers were combined. The solvent was removed under reduced pressure from 80 % of the solution. 35 mL of acetone were added to the residue and the solvent was removed under reduced pressure. The residue was redissolved in 19 mL of acetone. While stirring, 40 mL of $H_2O$ were added to this solution dropwise over a period of several minutes. Stirring was continued for another 30 min. The resulting suspension was filtered and the filter cake was washed with a mixture of 25 mL of $H_2O$ and 9 mL of acetone. The product was dried under reduced pressure at 35 °C for 9 h and left to equilibrate with air moisture to afford 3.032 g (NMR assay 90 %, overall yield 69 %) of compound (III).
HPLC purity: 92.43 area %

Example 9: Preparation of the compound of formula (III)

A suspension of 6.23 g of $Me_3SBr$ in 100 mL of THF was cooled to -22 °C and 8.01 g of potassium tert-butoxide were added in one portion. The temperature did not exceed -18° C. The mixture was stirred under a nitrogen atmosphere at -22 to -18 °C for another 2 h. A solution of 5.00 g of the compound of formula (II) (HPLC assay 97 %) in 100 mL of $CH_2Cl_2$ was prepared and cooled to -78 °C under a nitrogen atmosphere.

The THF suspension was cooled to -78 °C and the prepared $CH_2Cl_2$ solution was quickly added to the suspension while stirring under a nitrogen atmosphere. After additional 4 min of stirring at -78 °C, the reaction mixture was poured into a solution of $NH_4Cl$ (3.84 g) in $H_2O$ (31 mL. After stirring at room temperature for 45 min, the mixture was stored at -20 °C overnight. The layers were separated and the aqueous phase was washed with 2 x 8 mL $CH_2Cl_2$. The organic layers were combined.

The solvent was removed under reduced pressure from 80 % of the solution. 35 mL of acetone were added to the residue and the solvent was removed under reduced pressure. The residue was redissolved in 19 mL of acetone. While stirring, 40 mL of $H_2O$ were added to this solution dropwise over a period of several minutes. Stirring was continued for another 30 min. The resulting suspension was filtered and the filter cake was washed with a mixture of 25 mL of $H_2O$ and 9 mL of acetone. The product was dried under reduced pressure at 35 °C for 9 h and left to equilibrate with air moisture to afford 3.089 g (HPLC assay 91 %, overall yield 71 %) of the compound of formula (III).
HPLC purity: 93.60 area %

Example 10: Preparation of the compound of formula (III)

A suspension or 6.50 g of $Me_3SBF_4$ in 100 mL of THF was cooled to -25 °C and 2.50 g of potassium tert-butoxide were added in one portion. The temperature did not exceed -20 °C. The resulting mixture was stirred at -26 to -24 °C under a nitrogen atmosphere for another 2 h. A solution of 5.00 g of the compound of formula (II) (HPLC assay 95 %) in 100 mL of $CH_2Cl_2$ was prepared and cooled to -78 °C under a nitrogen atmosphere.

The THF suspension was cooled to -78 °C and the prepared $CH_2Cl_2$ solution was quickly added to the suspension while stirring under a nitrogen atmosphere. After additional 5 min of stirring at -78 °C, the reaction mixture was poured into a solution of $NH_4Cl$ (3.84 g) in $H_2O$ (31 mL). After stirring at room temperature for 55 min, the reaction mixture was stored at -20 °C overnight. The layers were separated and the aqueous phase was washed with 2 x 8 mL of $CH_2Cl_2$. The organic layers were combined. The solvent was removed under reduced pressure from 80 % of the solution. 35 mL of acetone were added to the residue and the solvent was removed under reduced pressure. Half of the residue was redissolved in 9 mL of acetone. While stirring, 20 mL of $H_2O$ were added to this solution dropwise over a period of several minutes. Stirring was continued for additional 30 min. The resulting suspension was filtered and the filter cake was washed with a mixture of 12.5 mL of $H_2O$ and 4.5 mL of acetone. The product was dried under reduced pressure at 35 °C for 9 h and left to equilibrate with air moisture to afford 1.394 g (HPLC assay 85 %, overall yield 61 %) of compound (III).
HPLC purity: 93.93 area %

Example 11: Preparation of the compound of formula (III)

A suspension of 6.50 g of $Me_3SBF_4$ in 100 mL of THF was cooled to -27 °C and 21 mL of 1 M solution of potassium hexamethyldisilazide in THF was added in one portion. The temperature did not exceed -22 °C. The resulting mixture was stirred at -26 to -24 °C under a nitrogen atmosphere for additional 2 h. A solution of 5.00 g of the compound of formula (II) (HPLC assay 95 %) in 100 mL of $CH_2Cl_2$ was prepared and cooled to -78 °C under a nitrogen atmospheres.

The THF suspension was cooled to -78 °C and the prepared $CH_2Cl_2$ solution was quickly added to the suspension while stirring under a nitrogen atmosphere. After additional 5 min of stirring at -78 °C, the reaction mixture was poured into a solution of $NH_4Cl$ (3.87 g) in $H_2O$ (31 mL). After stirring at room temperature for 1 h, the reaction mixture was stored at -20 °C overnight. The layers were separated and the aqueous phase was washed with 2 x 8 mL of $CH_2Cl_2$. The organic layers were combined. The solvent was removed under reduced pressure from 80 % of the solution. 35 mL of acetone were added to the residue and the solvent was removed under reduced pressure. The residue was redissolved in 19 mL of acetone. While stirring, 40 mL of $H_2O$ were added to this solution dropwise over a period of several minutes. Stirring was continued for additional 30 min. The resulting suspension was filtered and the filter cake was washed with a mixture of 25 mL of $H_2O$ and 9 mL of acetone. The product was dried under reduced pressure at 35 °C for 9 h and left to equilibrate with air moisture to afford 2.718 g (HPLC assay 86 %, overall yield 60 %) of the compound of formula (III). HPLC purity: 94.84 area %

Example 12: Preparation of the compound of formula (III)

4.10 g of *tert*-butanol were added to a suspension of 26.0 g of $Me_3SBF_4$ in 400 mL of THF. After cooling the resulting mixture to -25 °C, 10.0 g of potassium tert-butoxide were added in one portion. The temperature did not exceed -21 °C. The resulting mixture was stirred at -25 to -21 °C under a nitrogen atmosphere for additional 2 h. A solution of 20.0 g of the compound of formula (II) (HPLC assay 91 %) in 400 mL of $CH_2Cl_2$ was prepared and cooled to -78 °C under a nitrogen atmosphere. The THF suspension was cooled to -78 °C and the prepared $CH_2Cl_2$ solution was quickly added to the suspension while stirring under a nitrogen atmosphere. After additional 5 min of stirring at -78 °C, the reaction mixture was poured into a solution of $NH_4Cl$ (15.3 g) in $H_2O$ (124 mL). After stirring at room temperature for 1 h, the mixture was stored at -20 °C overnight.

The layers were separated and the aqueous phase was washed with 2 x 32 mL of $CH_2Cl_2$. The organic layers were combined and the solvent was removed under reduced pressure. 140 mL of acetone were added to the residue and the solvent was removed under reduced pressure. The residue was redissolved in 76 mL of acetone. While stirring, 160 mL of $H_2O$ were added to this solution dropwise over a period of 25 min. Stirring was continued for another 30 min. The resulting suspension was filtered and the filter cake was washed with a mixture of 100 mL of $H_2O$ and 36 mL of acetone. The product was dried under reduced pressure at 35 °C for 9 h and left to equilibrate with air moisture to afford 12.307 g (HPLC assay 87 %, yield 58 %) of the compound of formula (III).
HPLC purity: 94.97 area %

Example 13: Preparation of the compound of formula (III)

1.005 g of *tert*-butanol were added to a suspension of 3.887 g of $Me_3SBF_4$ in 100 mL of THF. After cooling the resulting mixture to -25 °C, 2.508 g of potassium tert-butoxide were added in one portion. The temperature did not exceed -20 °C. The resulting mixture was stirred at -25 to -21 °C under a nitrogen atmosphere for another 2 h. A solution of 6.254 g of the compound of formula (II) (HPLC assay 76 %) in 100 mL of $CH_2Cl_2$ was prepared and the solvent was removed under reduced pressure. The residue was redissolved in 100 mL of fresh $CH_2Cl_2$ and cooled to -78 °C under a nitrogen atmosphere.

The THF suspension was cooled to -78 °C and the prepared $CH_2Cl_2$ solution was added to the suspension over a period of 10 min while stirring under a nitrogen atmosphere with the temperature not exceeding -72 °C. After additional 20 min of stirring at -78 °C, the reaction mixture was poured into a solution of $NH_4Cl$ (3.842 g) in $H_2O$ (31 mL). After stirring at room temperature for 1 h, the layers were separated and the aqueous phase was washed with 2 x 8 mL of $CH_2Cl_2$. The organic layers were combined and the solvent was removed under reduced pressure. 40 mL of acetone were added to the residue and the solvent was removed under reduced pressure to obtain 6.390 g of residue. 6.175 g of the residue were redissolved in 21 mL of acetone. While stirring, 44 mL of $H_2O$ were added to this solution dropwise over a period of several minutes. Stirring was continued for another 30 min. The resulting suspension was filtered and the filter cake was washed with a mixture of 27 mL of $H_2O$ and 10 mL of acetone. The product was dried under reduced pressure at 35 °C for 6 h and left to equilibrate with air moisture to afford 3.401 g (HPLC assay 83 %, overall yield 60 %) of compound (III).
HPLC purity: 93.28 area %

Example 14: Preparation of the compound of the formula (III)

6.027 g of *tert*-butanol were added to a suspension of 23.330 g of $Me_3SBF_4$ in 600 mL of THF. After cooling the resulting mixture to -25 °C, 15.077 g of potassium tert-butoxide were added in one portion. The temperature did not exceed -20 °C. The resulting mixture was stirred at -25 to -21 °C under a nitrogen atmosphere for additional 2 h. A solution of 38.535 g of the compound of formula (II) (HPLC assay 74 %) in 600 mL of $CH_2Cl_2$ was prepared and the

solvent was removed under reduced pressure. The residue was redissolved in 600 mL of fresh $CH_2Cl_2$ and cooled to -78 °C under a nitrogen atmosphere.

The THF suspension was cooled to -78 °C and the prepared $CH_2Cl_2$ solution was added to the suspension over a period of 11 min while stirring under a nitrogen atmosphere with the temperature not exceeding -72 °C. After additional 20 min of stirring at -78 °C, the reaction mixture was poured into a solution of $NH_4Cl$ (23.1 g) in $H_2O$ (185 mL). After stirring at room temperature for 90 min, the layers were separated and the aqueous phase was washed with 2 x 50 mL of $CH_2Cl_2$. The organic layers were combined and the solvent was removed under reduced pressure. 240 mL of acetone were added to the residue and the solvent was removed under reduced pressure to obtain 38.537 g of residue. 37.524 g of the residue were dissolved in 140 mL of acetone. While stirring, 293 mL of $H_2O$ were added to this solution dropwise over a period of several minutes. Stirring was continued for another 30 min. The resulting suspension was filtered and the filter cake was washed with a mixture of 180 mL of $H_2O$ and 67 mL of acetone. The product was dried under reduced pressure at 35 °C for 6 h and left to equilibrate with air moisture to afford 20.691 g (HPLC assay 84 %, overall yield 62 %) of compound (III).

HPLC purity: 93.12 area %
XRPD: Figure 3.

Example 15: Preparation of tulathromycin phosphate (1:2)

A mixture of 11.5 g of the compound of formula (III) (HPLC assay 87 %), 60 mL of 2-propanol and 31 mL of *n*-propylamine was stirred at 55 °C for 31 h. Volatile components of the reaction mixture were removed under reduced pressure. 115 mL of ethanol and 9 mL of $H_2O$ were added to the residue. To the resulting mixture, a solution of orthophosphoric acid (3.53 g) in ethanol (52 mL) was added dropwise while stirring. The resulting suspension was left stirring overnight and filtered. The filter cake was washed with 75 mL of ethanol. The product was dried under reduced pressure at 35 °C for 6.5 h and left to equilibrate with air moisture to afford 14.372 g (HPLC assay 67 %, yield 72 %) of tulathromycin phosphate (1:2).
HPLC purity: 97.54 area %

Example 16: Preparation of tulathromycin phosphate (1:2)

A mixture of 5.746 g of the compound of formula (III) (HPLC assay 84 %), 30 mL of 1-propanol and 15.5 mL of *n*-propylamine was stirred at 56 °C for 31 h. Volatile components of the reaction mixture were removed under reduced pressure to obtain 6.391 g of residue. 57 mL of ethanol and 4.5 mL of $H_2O$ were added to 6.281 g of the residue. To the resulting mixture, a solution of orthophosphoric acid (1.339 g) in ethanol (26 mL) was added dropwise while stirring. The resulting suspension was left stirring overnight and filtered. The filter cake was washed with 35 mL of ethanol. The product was dried under reduced pressure at 35 °C for 7 h and left to equilibrate with air moisture to afford 5.906 g (HPLC assay 70 %, overall yield 65 %) of tulathromycin phosphate (1:2).

HPLC purity: 97.34 area %
XRPD: Figure 4.

Example 17: Preparation of tulathromycin

A solution of 15.138 g of $K_2CO_3$ in 230 mL of $H_2O$ was added to a suspension of 34.92 g of tulathromycin phosphate (1:2) (HPLC assay 72 %) in 230 mL of $CH_2Cl_2$. The layers were separated and the aqueous phase was washed with 2 x 50 mL $CH_2Cl_2$. The solvent was removed under reduced pressure from 98 % of the combined organic layers. 50 mL of methyl ethyl ketone were added to the residue and the solvent was again removed under reduced pressure to obtain 24 g of residue. 20 g of the residue and 25 mL of methyl ethyl ketone were charged into a glass reactor, equipped with a condenser and a mechanical stirrer. The mixture was heated to 80 °C and stirred until a clear solution was formed. Next, 75 mL of *n*-heptane with a temperature of 35 °C were added to the solution. The solution was maintained by means of a thermostat at 50 °C. At this temperature of solution, the reactor jacket temperature was set to 50 °C and vacuum was applied to distill off approx. 60 % of the solvents. During vacuum distillation, the temperature of the reactor content was not lower than 30 °C, mainly between 35 - 40 °C. A suspension was formed during the distilation phase. After the distillation was completed, 100 mL of *n*-heptane with a temperature of 35 °C were added and the mixture was further stirred for the next 30 min (the reactor jacket temperature was set to 40 °C and the temperature of reactor content was 38 °C). The obtained suspension was cooled with an average cooling rate of 0.10 °C/min to the final crystallization temperature, 0 °C. After the crystallization mixture was cooled to 0 °C, it was further maintained at this temperature for the next 12 h. The obtained product was isolated with suction filtration using a Büchner funnel and

nitrogen atmosphere. The isolated product was dried at 40 °C in a vacuum dryer under reduced pressure (< 100 mbar) for 24 h to afford 11.85 g of tulathromycin (HPLC assay 98 %, overall yield 71 %).
HPLC purity: 93.16 area %

Example 18: Preparation of tulathromycin

A solution of 5.83 g of $K_2CO_3$ in 19 mL of $H_2O$ was added to a suspension of 13.5 g of tulathromycin phosphate (1:2) (HPLC assay 67 %) in 52 mL of $CH_2Cl_2$. The layers were separated and the aqueous phase was washed with 2 x 15 mL of $CH_2Cl_2$. 95 % of the combined organic layers were concentrated in a glass reactor using vacuum distillation until an oily residue was obtained (9.58 g). Methyl ethyl ketone (12.5 mL) was charged into a glass reactor, equipped with a condenser and a mechanical stirrer. The mixture was heated to 80 °C and stirred until a clear solution was formed. Then, 37 mL of *n*-heptane with a temperature of 35 °C were added to the solution. The solution was maintained by means of a thermostat at 50 °C. At this temperature of solution, the reactor jacket temperature was set to 50 °C and vacuum was applied to distil off approx. 60 vol. % of the solvents. During vacuum distillation, the temperature of the reactor content was not lower than 30 °C, mainly between 35 - 40 °C. A suspension was formed during the distillation phase. Furthermore, after the distillation phase was completed, 50 mL of *n*-heptane with a temperature of 35 °C were added and the mixture was further stirred for the next 30 min (the reactor jacket temperature was set to 40 °C). The obtained suspension was cooled with an average cooling rate of 0.10 °C/min to the final crystallization temperature, 0 °C. After the crystallization mixture was cooled to 0 °C, it was further maintained at this temperature for the next 12 h. The obtained product was isolated with suction filtration using a Büchner funnel and nitrogen atmosphere. The isolated product was dried at 40 °C in a vacuum dryer under reduced pressure (< 100 mbar) for 24 h to afford 5.21 g of tulathromycin (HPLC assay 100 %, overall yield 75 %).

HPLC purity: 99.07 area %
Elemental analysis: calculated for $C_{41}H_{79}N_3O_{12}$: C, 61.09; H, 9.88; N, 5.21; O, 23.82, found: C, 61.28; H, 10.24; N, 5.17.

Example 19: Crystallization of tulathromycin

Diisopropyl ether (1450 mL) and tulathromycin base (86.7 g, HPLC purity 94.8 area %) were charged into a glass reactor, equipped with a condenser and a mechanical stirrer. The mixture was heated with an applied jacket temperature of 70 °C, until a clear solution was formed. The solution was cooled to a temperature of 45 °C and seeded with 0.87 g of tulathromycin base crystals (anhydrous form). After seeding the reactor jacket temperature was set to 45 °C and vacuum was applied to distill off the solvent until the volume of the suspension reached 230 mL. Furthermore, after the distillation phase was completed, 213 mL of *n*-heptane with a temperature of 40 °C were added in the next 10 min (the reactor jacket temperature was maintained at 45 °C). The obtained suspension was cooled with an average cooling rate of 0.4 °C/min to the final crystallization temperature, 5 °C. After the crystallization mixture was cooled to 5 °C, it was further maintained at this temperature for the next 17 h. The obtained product was isolated with suction filtration using a Büchner funnel and nitrogen atmosphere and washed with 50 mL of ice-cold *n*-heptane.
The isolated product was dried at 40 °C in a vacuum dryer under reduced pressure (< 100 mbar) for 24 h to afford 60.3 g of tulathromycin base in an anhydrous form.
HPLC purity: 97.6 area %

Example 20: Crystallization of tulathromycin

Diisopropyl ether (1000 mL) and tulathromycin base (60.0 g, HPLC purity 95.8 area %) were charged into a glass reactor, equipped with a condenser and a mechanical stirrer. The mixture was heated with an applied jacket temperature of 70 °C, until a clear solution was formed. The solution was cooled to a temperature of 40 °C and seeded with 0.60 g of tulathromycin base crystals (anhydrous form). After seeding the reactor jacket temperature was set to 40 °C and vacuum was applied to distill off the solvent until the volume of the suspension reached 150 mL. Furthermore, after the distillation phase was completed, 150 mL of *n*-heptane with a temperature of 40 °C were added in the next 10 min (the reactor jacket temperature was maintained at 45 °C). The obtained suspension was cooled with an average cooling rate of 0.4 °C/min to the final crystallization temperature, 5 °C. After the crystallization mixture was cooled to 5 °C, it was further maintained at this temperature for the next 17 h. The obtained product was isolated with suction filtration using a Büchner funnel and nitrogen atmosphere and washed with 60 mL of ice-cold *n*-heptane.
The isolated product was dried at 50 °C in a vacuum dryer under reduced pressure (< 100 mbar) for 24 h to afford 41.1 g of tulathromycin base in an anhydrous form.
HPLC purity: 97.1 area %

Example 21: Preparation of tulathromycin phosphate (1:2) and tulathromycin

A mixture of 4.000 g of the compound of formula (III) (HPLC assay 87 %), 21 mL of acetonitrile and 10.7 mL of n-propylamine was stirred at about 47 °C for about 25 h. The reaction mixture was divided into 2 equal parts and volatile components were removed under reduced pressure to obtain 2.102 g and 2.095 g of residue, respectively.

(i) 40 mL of ethanol and 3.1 mL of $H_2O$ were added to 98 % of the first residue. To the resulting mixture, a solution of orthophosphoric acid (0.608 g) in ethanol (8.9 mL) was added dropwise while stirring. The resulting suspension was left stirring overnight and filtered. The filter cake was washed with 6 mL of ethanol. The product was dried under reduced pressure at 35 °C for 9 h and left to equilibrate with air moisture to afford 2.132 g (HPLC assay 60 %, overall yield 56 %) of tulathromycin phosphate (1:2).
HPLC purity: 63.76 area %
(ii) 11 mL of $CH_2Cl_2$ and 28 mL of *n*-heptane were added to 98 % of the second residue. The resulting mixture was evaporated under reduced pressure to about 20 % of the initial volume. The resulting mixture was stirred at RT for about 3.5 h and filtered. The filter cake was washed with 5 mL of *n*-heptane. The product was dried under reduced pressure at 35 °C for 9 h to afford 0.400 g (HPLC assay 89 %, overall yield 19 %) of tulathromycin.
HPLC purity: 89.30 area %

Example 22: Preparation of tulathromycin phosphate (1:2) and tulathromycin

A mixture of 4.000 g of the compound of formula (III) (HPLC assay 87 %) and 10.7 mL of *n*-propylamine was stirred at about 45 °C for about 26 h.

(i) Volatile components were removed from about 50 % of the reaction mixture under reduced pressure to obtain 2.016 g of residue. 40 mL of ethanol and 3.1 mL of $H_2O$ were added to 98 % of the residue. To the resulting mixture, a solution of orthophosphoric acid (0.607 g) in ethanol (8.9 mL) was added dropwise while stirring. The resulting suspension was left stirring overnight and filtered. The filter cake was washed with 6 mL of ethanol. The product was dried under reduced pressure at 35 °C for 9 h and left to equilibrate with air moisture to afford 1.986 g (HPLC assay 67 %, overall yield 58 %) of tulathromycin phosphate (1:2).
HPLC purity: 76.97 area %
(ii) Volatile components were removed from about 46 % of the reaction mixture under reduced pressure to obtain 1.897 g of residue. 11 mL of $CH_2Cl_2$ and 28 mL of *n*-heptane were added to 97 % of the second residue. The resulting mixture was evaporated under reduced pressure to about 30 % of the initial volume. The resulting mixture was stirred at RT overnight and filtered. The filter cake was washed with 5 mL of *n*-heptane. The product was dried under reduced pressure at 35 °C for 9 h to afford 0.312 g (HPLC assay 95 %, HPLC purity: 88.51 area %) of tulathromycin.

The filtrate was evaporated under reduced pressure to about one half of the initial volume. The resulting mixture was stirred at RT for about 2.5 h and filtered. The filter cake was washed with 5 mL of *n*-heptane. The product was dried under reduced pressure at 35 °C for 9 h to afford 0.312 g (HPLC assay 90 %, HPLC purity: 89.91 area %) of tulathromycin. Overall yield: 34 %.

Example 23: Preparation of tulathromycin

A mixture of 1.000 g of the compound of formula (III) (HPLC assay 87 %), 5.2 mL of 2-propanol and 1.920 g of *n*-propylamine was stirred at about 50 °C for about 29 h. Volatile components were removed from about 99 % of the reaction mixture under reduced pressure to obtain 1.047 g (HPLC assay 76 %, overall yield 86 %) of crude tulathromycin.
HPLC purity: 81.34 area %

Example 24: Preparation of tulathromycin

A mixture of 0.500 g of the compound of formula (III) (HPLC assay 87 %), 2.6 mL of *n*-heptane and 0.960 g of *n*-propylamine was stirred in a closed vial at about 70 °C for about 28 h. Volatile components were removed from about 99 % of the reaction mixture under reduced pressure to obtain 0.503 g (HPLC assay 64 %, overall yield 69 %) of crude tulathromycin.
HPLC purity: 69.08 area %

Example 25: Preparation of tulathromycin

A mixture of 0.500 g of the compound of formula (III) (HPLC assay 83 %), 2.5 mL of 1-butanol and 0.916 g of *n*-propylamine was stirred at about 50 °C for about 23.5 h. Volatile components were removed from about 99 % of the reaction mixture under reduced pressure to obtain 0.825 g (HPLC assay 42 %, overall yield 78 %) of crude tulathromycin. HPLC purity: 80.15 area %

Example 26: Preparation of tulathromycin

A mixture of 0.500 g of the compound of formula (III) (HPLC assay 83 %), 2.5 mL of 2-butanol and 0.916 g of *n*-propylamine was stirred at about 50 °C for about 23 h. Volatile components were removed from about 99 % of the reaction mixture under reduced pressure to obtain 0.512 g (HPLC assay 61 %, overall yield 70 %) of crude tulathromycin. HPLC purity: 81.75 area %

Example 27: Preparation of tulathromycin

A mixture of 0.500 g of the compound of formula (III) (HPLC assay 83 %), 2.5 mL of *i*-butanol and 0.916 g of *n*-propylamine was stirred at about 50 °C for about 22 h. Volatile components were removed from about 99 % of the reaction mixture under reduced pressure to obtain 0.954 g (HPLC assay 38 %, overall yield 82 %) of crude tulathromycin. HPLC purity: 82.89 area %

Example 28: Preparation of tulathromycin

A mixture of 0.500 g of the compound of formula (III) (HPLC assay 83 %), 2.5 mL of 1-propanol and 0.916 g of *n*-propylamine was stirred at about 50 °C for about 22 h. Volatile components were removed from about 99 % of the reaction mixture under reduced pressure to obtain 0.548 g (HPLC assay 56 %, overall yield 69 %) of crude tulathromycin. HPLC purity: 76.39 area %

Example 29: Preparation of tulathromycin

A mixture of 0.500 g of the compound of formula (III) (HPLC assay 83 %), 2.5 mL of 2-methyl-2-butanol and 0.916 g of *n*-propylamine was stirred at about 50 °C for about 22 h. Volatile components were removed from about 99 % of the reaction mixture under reduced pressure to obtain 0.695 g (HPLC assay 40 %, overall yield 63 %) of crude tulathromycin.
HPLC purity: 68.20 area %

Example 30: Preparation of tulathromycin

A mixture of 0.500 g of the compound of formula (III) (HPLC assay 83 %), 2.627 g of *n*-propylamine and 0.365 mL of $H_2O$ was stirred at about 47 °C for about 21.5 h. Volatile components were removed from about 99 % of the reaction mixture under reduced pressure to obtain 0.509 g (HPLC assay 78 %, overall yield 90 %) of crude tulathromycin. HPLC purity: 81.83 area %

## Claims

1. A process for preparation of the compound of formula (II) wherein the compound of formula (I) is directly converted to the compound of formula (II):

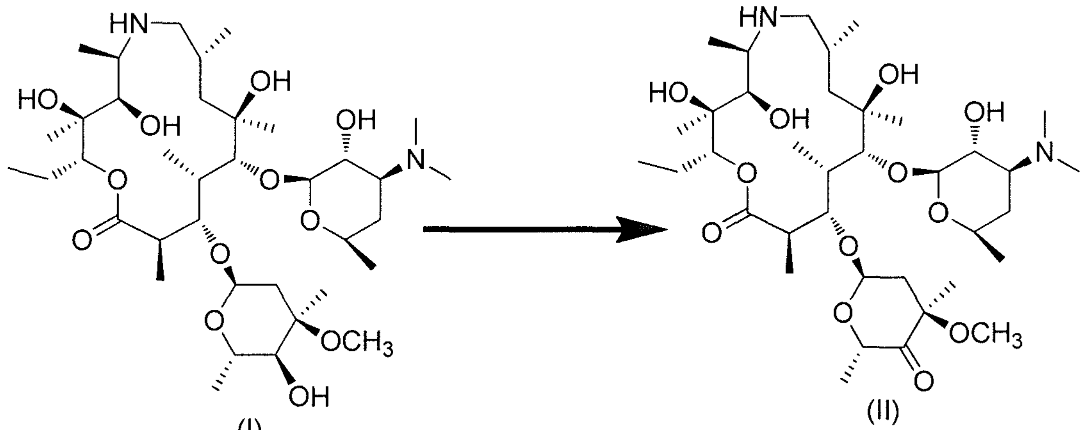

(I) (II),

wherein the conversion is performed with an oxidant and wherein the conversion is performed in the presence of an activating agent,
the oxidant being dimethyl sulfoxide, and the activating agent being trifluoroacetic anhydride.

2. The process according to claim 1, wherein the compound of formula (I) is converted to the compound of formula (II) without prior protection of C-2' hydroxyl group or any other functional group of the compound of formula (I)

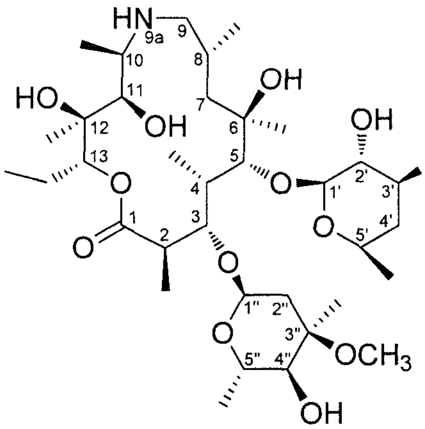

(I).

3. The process according to any of claims 1-2, wherein the conversion is performed in the presence of a base, optionally wherein the base is an organic amine.

4. The process according to claim 3, wherein the base is triethylamine.

5. The process according to any of claims 1-4, wherein the conversion is performed in a solvent which has a melting point below -80 °C, optionally
wherein the solvent is dichloromethane, toluene, tetrahydrofuran, 2-methyltetrahydrofuran, diethyl ether, and/or acetone.

6. The process according to claim 5, wherein the solvent is dichloromethane.

7. The process according to claim 1, wherein the conversion is performed with dimethyl sulfoxide, trifluoroacetic anhydride and triethylamine in dichloromethane at -80 to -30 °C.

8. The process according to any of the preceding claims, further comprising conversion of the compound of formula (II) to tulathromycin.

9. The process of any of claims 1 to 7, wherein purification of the compound of formula (II) comprises

a) suspending the compound of formula (II) in acetone at a predetermined temperature and stirring the suspension for a certain time; or

b) crystallizing the compound of formula (II) from a system consisting of $CH_2Cl_2$ and diethyl ether.

**10.** The process according to claim 9, wherein the predetermined temperature is in the range from about - 20 °C to about 56 °C and the time for stirring is in the range from about 10 min to about 24 h.

**11.** The process according to any of claims 1 to 7, 9 and 10, which process further comprises preparing tulathromycin or a pharmaceutically acceptable salt or hydrate thereof from the compound of formula (II).

**12.** The process according to any of claims 1 to 7 or 9 to 11, which process further comprises:

converting the compound of formula (II) into a compound of formula (III) or a salt or hydrate thereof,

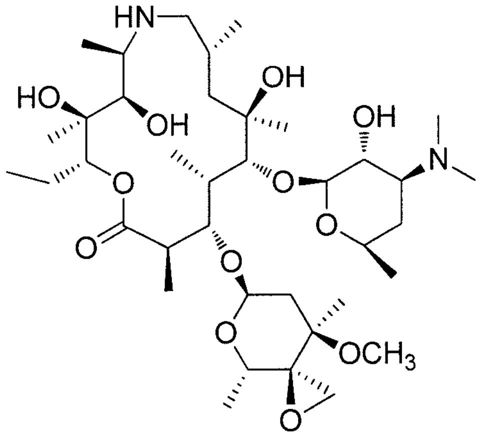

**(III)**

and optionally converting the compound of formula (III) or a salt or hydrate thereof to tulathromycin or a pharmaceutically acceptable salt or hydrate thereof.

## Patentansprüche

**1.** Verfahren zur Herstellung der Verbindung der Formel (II), wobei die Verbindung der Formel (I) direkt in die Verbindung der Formel (II) umgewandelt wird:

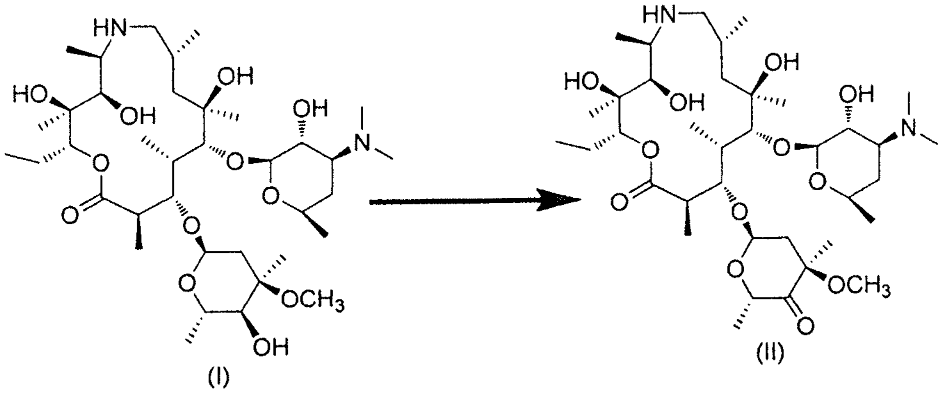

(I) (II)

,

wobei die Umwandlung mit einem Oxidans durchgeführt wird und wobei die Umwandlung in der Gegenwart eines aktivierenden Mittels durchgeführt wird, wobei das Oxidans Dimethylsulfoxid ist und das aktivierende Mittel Trifluoressigsäureanhydrid ist.

**2.** Verfahren gemäß Anspruch 1, wobei die Verbindung der Formel (I) in die Verbindung der Formel (II) ohne vorher

Schützen der Hydroxylgruppe C-2' oder von jedweder anderen funktionellen Gruppe der Verbindung der Formel (I) umgewandelt wird

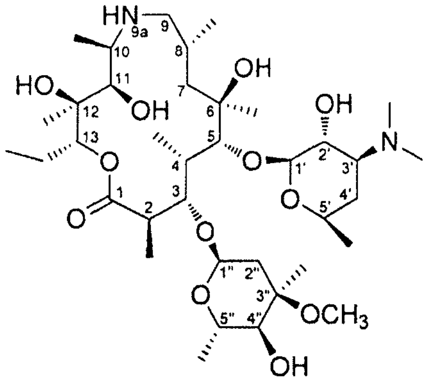

(I).

3. Verfahren gemäß einem der Ansprüche 1-2, wobei die Umwandlung in der Gegenwart einer Base durchgeführt wird, wobei die Base gegebenenfalls ein organisches Amin ist.

4. Verfahren gemäß Anspruch 3, wobei die Base Triethylamin ist.

5. Verfahren gemäß einem der Ansprüche 1-4, wobei die Umwandlung in einem Lösungsmittel, welches einen Schmelzpunkt von unter -80°C aufweist, durchgeführt wird, wobei das Lösungsmittel gegebenenfalls Dichlormethan, Toluol, Tetrahydrofuran, 2-Methyltetrahydrofuran, Diethylether und/oder Aceton ist.

6. Verfahren gemäß Anspruch 5, wobei das Lösungsmittel Dichlormethan ist.

7. Verfahren gemäß Anspruch 1, wobei die Umwandlung mit Dimethylsulfoxid, Trifluoressigsäureanhydrid und Triethylamin in Dichlormethan bei -80 bis -30°C durchgeführt wird.

8. Verfahren gemäß einem der vorhergehenden Ansprüche, ferner umfassend Umwandeln der Verbindung der Formel (II) in Tulathromycin.

9. Verfahren nach einem der Ansprüche 1 bis 7, wobei eine Reinigung der Verbindung der Formel (II) umfasst

a) Suspendieren der Verbindung der Formel (II) in Aceton bei einer vorher bestimmten Temperatur und Rühren der Suspension für eine bestimmte Zeit; oder
b) Kristallisieren der Verbindung der Formel (II) in einem System, welches aus $CH_2Cl_2$ und Diethylether besteht.

10. Verfahren gemäß Anspruch 9, wobei die vorher bestimmte Temperatur im Bereich von etwa -20°C bis etwa 56°C liegt und die Zeit zum Rühren im Bereich von etwa 10 min bis etwa 24 h liegt.

11. Verfahren gemäß einem der Ansprüche 1 bis 7, 9 und 10, wobei das Verfahren ferner umfasst
Herstellen von Tulathromycin oder eines pharmazeutisch verträglichen Salzes oder Hydrates davon aus der Verbindung der Formel (II).

12. Verfahren gemäß einem der Ansprüche 1 bis 7 oder 9 bis 11, wobei das Verfahren ferner umfasst:

Umwandeln der Verbindung der Formel (II) in eine Verbindung der Formel (III) oder ein Salz oder Hydrat davon,

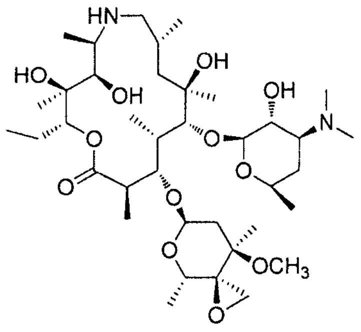

(III)

und gegebenenfalls Umwandeln der Verbindung der Formel (III) oder von einem Salz oder Hydrat davon in Tulathromycin oder ein pharmazeutisch verträgliches Salz oder Hydrat davon.

## Revendications

1. Un procédé de préparation du composé de formule (II) dans lequel le composé de formule (I) est directement converti dans le composé de formule (II) :

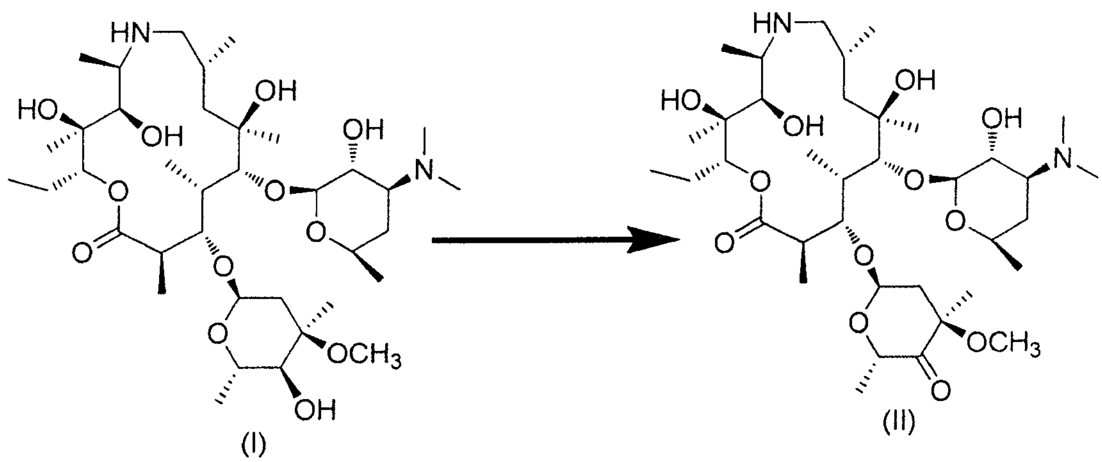

(I) (II)

.

dans lequel la conversion est effectuée avec un oxydant et dans lequel la conversion est effectuée en présence d'un agent activant, l'oxydant étant le diméthylsulfoxyde, et l'agent activant étant l'anhydride trifluoroacétique.

2. Le procédé selon la revendication 1, dans lequel le composé de formule (I) est converti dans le composé de formule (II) sans protection préalable du groupe hydroxyle C-2' ou de tout autre groupe fonctionnel du composé de formule (I)

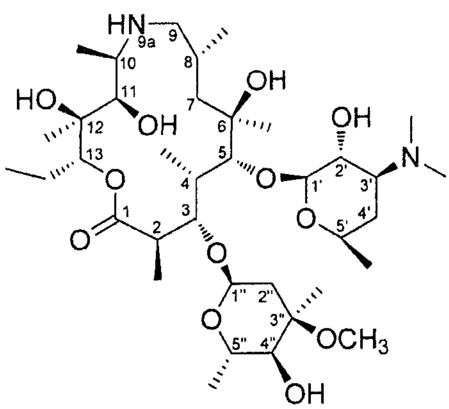

(I) .

3. Le procédé selon l'une quelconque des revendications 1 à 2, dans lequel la conversion est effectuée en présence

d'une base, facultativement
dans lequel la base est une amine organique.

**4.** Le procédé selon la revendication 3, dans lequel la base est la triéthylamine.

**5.** Le procédé selon l'une quelconque des revendications 1 à 4, dans lequel la conversion est effectuée dans un solvant qui présente un point de fusion inférieur à -80°C, facultativement
dans lequel le solvant est le dichlorométhane, le toluène, le tétrahydrofurane, le 2-méthyltétrahydrofurane, l'éther diéthylique et/ou l'acétone.

**6.** Le procédé selon la revendication 5, dans lequel le solvant est le dichlorométhane.

**7.** Le procédé selon la revendication 1, dans lequel la conversion est effectuée avec le diméthylsulfoxyde, l'anhydride trifluoroacétique et la triéthylamine dans du dichlorométhane entre -80 °C et -30 °C.

**8.** Le procédé selon l'une quelconque des revendications précédentes, comprenant en outre la conversion du composé de formule (II) en tulathromycine.

**9.** Le procédé selon l'une quelconque des revendications 1 à 7, dans lequel la purification du composé de formule (II) comprend :

a) la mise en suspension du composé de formule (II) dans de l'acétone à une température prédéterminée et l'agitation de la suspension pendant un certain temps ; ou
b) la cristallisation du composé de formule (II) à partir d'un système se composant de $CH_2Cl_2$ et d'éther diéthylique.

**10.** Le procédé selon la revendication 9, dans lequel la température prédéterminée se situe dans l'intervalle d'environ -20 °C à environ 56 °C et la durée de l'agitation est comprise dans l'intervalle d'environ 10 minutes à environ 24 heures.

**11.** Le procédé selon l'une quelconque des revendications 1 à 7, 9 et 10, lequel procédé comprend en outre
la préparation de tulathromycine ou d'un sel ou d'un hydrate de celle-ci acceptable sur le plan pharmaceutique à partir du composé de formule (II).

**12.** Le procédé selon l'une quelconque des revendications 1 à 7 ou 9 à 11, lequel procédé comprend en outre
la conversion du composé de formule (II) dans un composé de formule (III) ou un sel ou un hydrate de celui-ci,

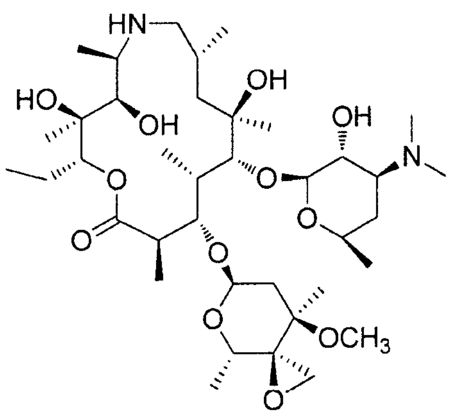

(III)

et facultativement la conversion du composé de formule (III) ou d'un sel ou d'un hydrate de celui-ci en tulathromycine ou un sel ou un hydrate de celle-ci acceptable sur le plan pharmaceutique.

FIGURE 1

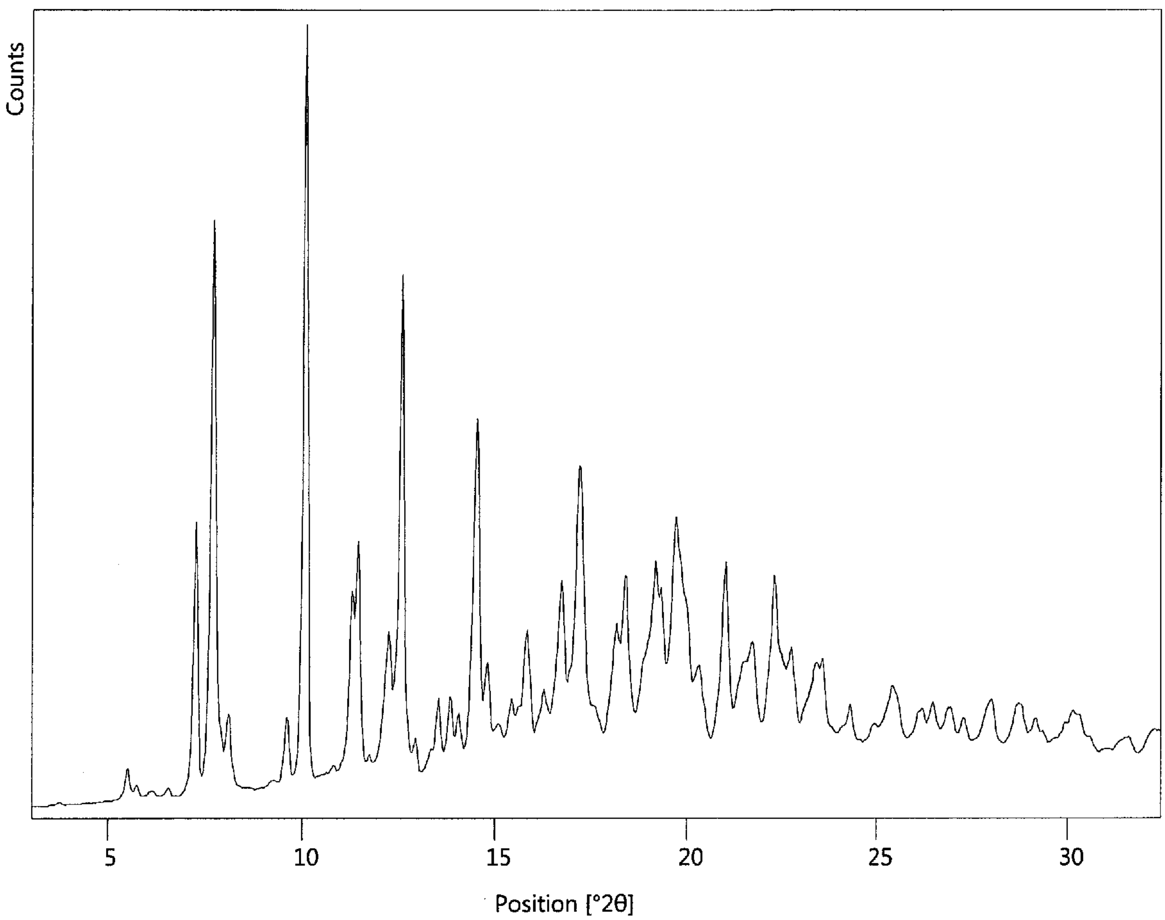

FIGURE 2

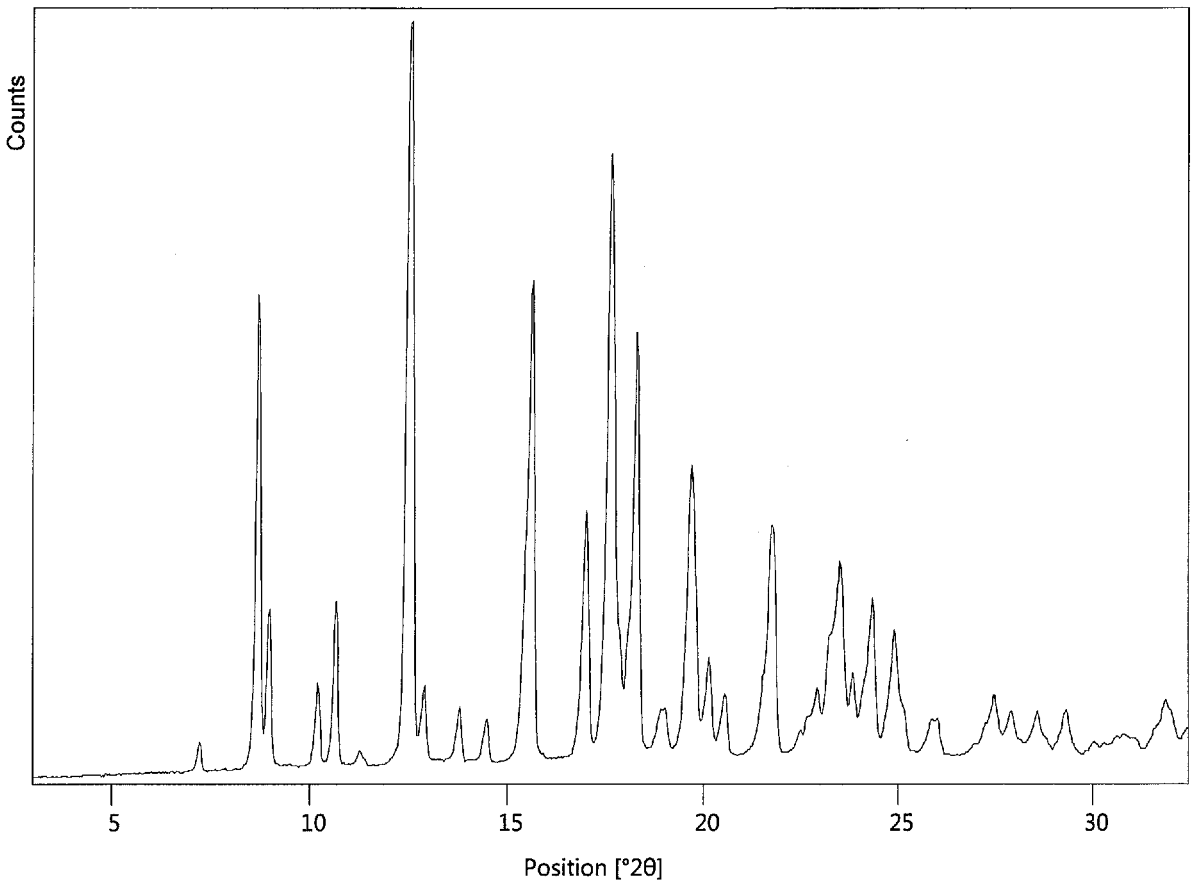

FIGURE 3

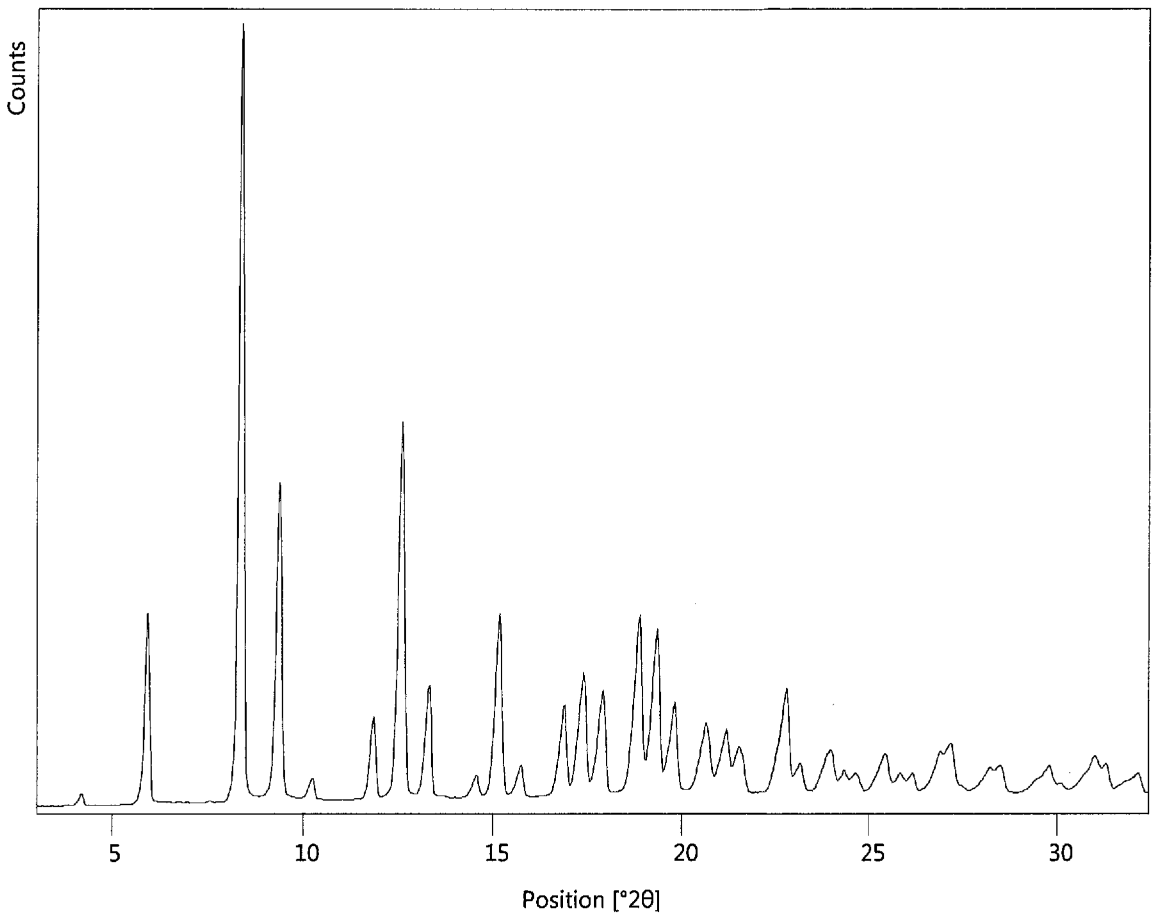

FIGURE 4

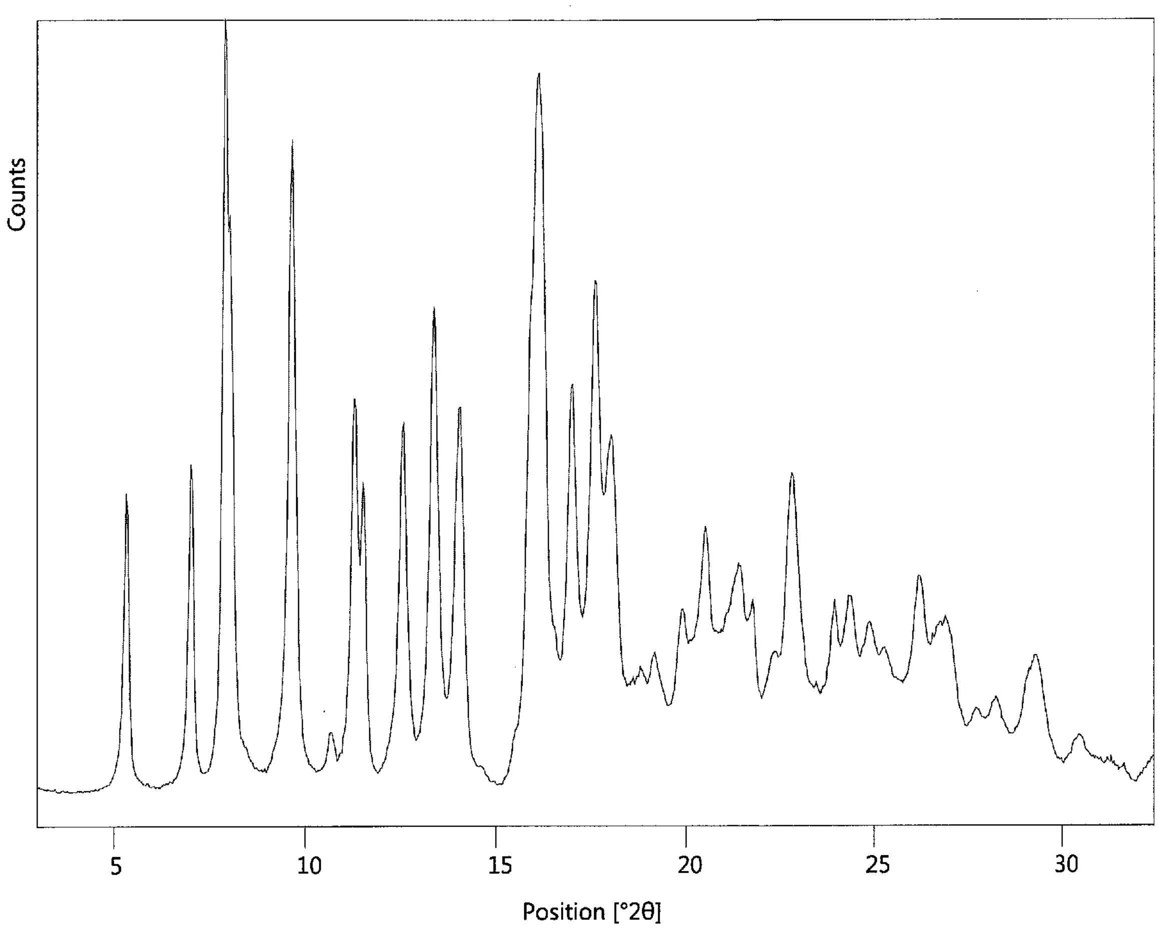

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 988310 A
- EP 1131331 A
- WO 02088158 A
- EP 1253153 A
- WO 2012038372 A
- CN 102295672 A
- CN 102260306 A
- CN 102786569 A
- CN 101648983 A
- GB 2047247 A
- EP 109253 A
- EP 136831 A
- HR 9300886
- EP 259789 A
- EP 827965 A
- EP 879823 A
- WO 9902541 A
- RU 2144924
- WO 0100640 A
- WO 02009640 A
- WO 0244193 A
- WO 03102009 A
- WO 0215842 A
- WO 2007029266 A
- WO 2007017898 A
- WO 0069874 A
- WO 0102414 A
- EP 2402355 A
- WO 2013013834 A
- WO 0155158 A
- WO 03011266 A
- CN 101647808
- CN 101933935
- WO 2012001089 A


### Non-patent literature cited in the description


- **LETAVIC.** *Bioorg. Med. Chem. Lett.,* 2002, vol. 12, 2771-2774
- **BRONK.** *Bioorg. Med. Chem. Lett.,* 2003, vol. 13, 1955-1958
- **WANG.** *Applied Chemical Industry,* 2012, 1294-1296
- **XIA.** *Editorial Office of Fine Chemicals,* 2012, 795-799
- **XIA.** Improving Process Of Synthesis Of Tulathromycin And Research Of Asymmetric Beckmann Rearranangement Reaction. *Thesis,* 2013
- **THOMAS T. TIDWELL.** Oxidation of Alcohols by Activated Dimethyl Sulfoxide and Related Reactions: An Update. *SYNTHESIS,* 10 October 1990
- **MARUŠIĆ IŠUK, Z. et al.** *Bioorg. Med. Chem.,* 2007, vol. 15, 4498-4510